# EUROPEAN PATENT SPECIFICATION

(11) **EP 2 816 970 B1**
(45) Date of publication and mention of the grant of the patent: **26.06.2019**
(21) Application number: 13752080.5
(22) Date of filing: 20.02.2013
(51) Int. Cl.: A61F 2/04, A61M 39/00

(54) **A METHOD AND APPARATUS FOR A CLOG RESISTANT ORIFICE**
VERFAHREN UND VORRICHTUNG FÜR EINE VERSTOPFUNGSSICHERE ÖFFNUNG
MÉTHODE ET APPAREIL POUR ORIFICE RÉSISTANT AUX CAILLOTS

(30) Priority: 21.02.2012 US 201261633999 P
(43) Date of publication of application: 31.12.2014
(73) Proprietor: Hospi Corporation, Newark, CA 94560 (US)
(72) Inventor: MACY, Bradford, Jr., Concord, CA 94520 (US)
(74) Representative: Clark, Jane Anne
(86) International application number: PCT/US2013/026951
(87) International publication number: WO 2013/126456

(56) References cited:
- EP-A1- 2 574 364
- EP-A2- 1 348 372
- WO-A1-87/02255
- WO-A1-2008/092013
- WO-A1-2009/058459
- WO-A1-2012/009187
- WO-A2-2006/078490
- US-A- 2 972 779
- US-A- 5 578 006
- US-A1- 2004 030 220
- US-A1- 2006 161 232
- US-A1- 2011 130 745

## Description

### CROSS-REFERENCE

This application claims the benefit of U.S. Provisional Application No. 61/633,999, filed February 21, 2012.

### BACKGROUND OF THE INVENTION

The present disclosure relates to medical devices, systems, and methods. In particular, improved medical devices, systems, and methods are provided to improve resistance against the clogging of the various ports or orifices in the various medical devices.

Medical tubes used for removal or delivery of a substance into or out of a body cavity typically have some form of transmission holes near the end of the tube, which transmit the particular substance into or out of the body cavity. Many tubes used such purposes clog frequently. Body secretions, bacterial biofilm, or accumulating residue can tend to build up around these holes or orifices causing clogging and subsequent malfunction of the device. Theoretically, the larger the hole the less it is able to clog. But, orifice size for many medical devices is often limited depending on the size of the tube and its utility. In order to create a clog resistant orifice, other design components may need to be taken into account. An orifice design which can exploit both the physics of the forces acting on a clogged orifice, and on the tube structure immediately surrounding the orifice may be highly desirable. The designs of existing transmission holes on medical tubes often do not take into account these concerns. Thus, clogs within medical tubes are all too frequent.

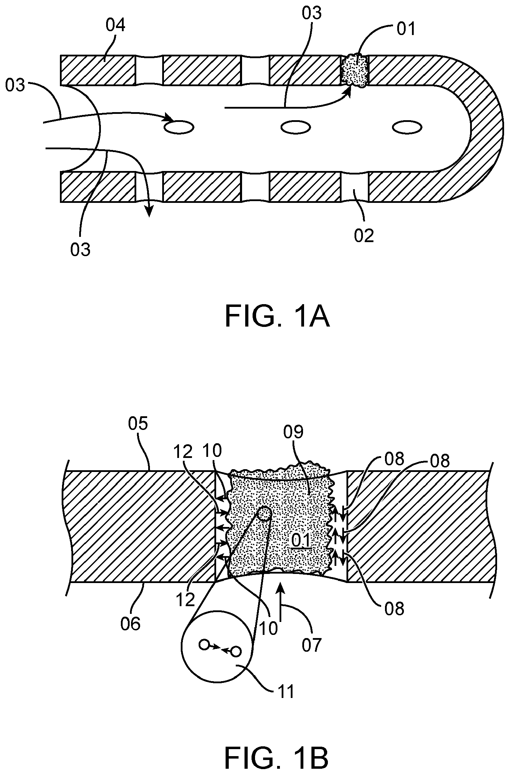

Figure 1A is an illustration of transmission orifices or holes typically present in many current medical tubes or other devices. The transmission orifices or holes are cut perpendicular to the surface of the lumen and outer wall of the tube. Figure 1A shows the tip of a typical tube with several orifices or holes **01** or **02** in the tube wall **04.** In Figure 1A, a hole **01** is clogged. Fluid is being pressured into the lumen to irrigate the tube as shown by arrows **03,** and the fluid is going out the open holes **02,** and applying a pressure to the clogged hole **01.** Figure 1B shows an enlarged view of the hole **01.** Fluid pressure is applying a force, represented by pressure force arrow **07,** to the clog in the direction indicated by the arrow **07.** Fluid cannot pass through the hole **01** from the inner lumen surface **06** to the outer tube surface **05** due to the clog. Figure 1B demonstrates the different forces at work within a clog **09,** the orifice **01,** and the wall of the tubing **04** which defines the orifice. Each of the various arrows represents force vectors. For reference, these forces are (i) the pressure force **07** exerted by the irrigation fluid, (ii) the adhesive force **08** between the wall and the clog, (iii) the tension force **10** from compaction of the clogged substance into the hole, the compression force **12** from the stretching of the tube wall and the retraction against the clog, and (iv) the cohesive force **11** which is the interaction between like particles of the clogged substance which forms the clog.

As shown in Figure 1B, the device is clogged, and attempts are made to clear the clog by irrigating the tube with a fluid. The force of the fluid attempts to push the clog out by applying a pressure force in the direction of the clog **07.** The clog is held together primarily by cohesive forces **11** of the particles making up the clog. These particles maybe stool, mucous, biofilm, etc. Along the wall of the clogged hole, adhesive forces **08** of the substance to the wall work against the force of the fluid through a shearing force between the wall and the clog. In addition, if the clog has been under any type of pressure, tension forces **10** of the clog against the wall also work against the fluid force **07.** An example of the creation of tension forces would be in the rectum where stool can be compressed into the holes of a tube by forces of the rectal muscle, creating an orifice which is tightly packed with stool.

Lastly, if the tube is flexible, forces of compression from the tube against the clog also come into play. More simply stated, the clog becomes packed into the hole with only a small open area in which the force of fluid can act against the forces holding the clog in place. These tension and compression forces can be much larger than the adhesive forces holding the clog to the wall, or the cohesive forces forming the clog in the first place. They can easily make the difference between being able to clear a clog with an irrigation fluid and being unable to clear the clog. An orifice that does not allow for the buildup of compression and tension forces would be highly superior to currently known and used transmission holes.

US2011/0130745, discloses a medical tube according to the preamble of claim 1, wherein it describes a catheter having a catheter body with a lumen in a distal lumen opening. A hole is formed through a wall of a distal portion of the catheter body. A portion of the proximal surface of the hole is inclined at an acute angle with respect to the longitudinal axis of the catheter body. The acute angle of the proximal hole surface opens proximally with respect to the catheter body.

US2006/0161232 describes a phase-change particulate ice slurry coolant medical delivery tubing and insertion device in which interfaces between various components that form the slurry flow path are configured so that there is no sudden reduction in the cross sectional area of the flow path and any narrowing of the flow path occurs in a gradual tapered manner.

WO2012/009187 describes a catheter having a catheter body with a lumen and a distal lumen opening. The catheter's lumen extends through the catheter body along a longitudinal axis of the catheter body. A hole is formed through a wall of a distal portion of the catheter body. A portion of the proximal surface of the hole is inclined at an acute angle with respect to the longitudinal axis of the catheter body. The acute angle of the proximal hole surface opens approximately with respect to the catheter body.

US2,972,779 describes a plastic tubing process to provide a plastic tubing product or catheter which has a smooth hole.

WO87/02255 describes an ultra sonic self-cleaning catheter system in which vibration is conveyed to the proximal orifices of an in dwelling catheter to disintegrate accumulated clogging deposits, large suspended particles and contaminating bacteria, viruses, fungi etc. Orifices may be recessed, hooded or enclosed and in some cases the capacator tip should be of absorptive material to deter propagation of the vibration to parts of the patient's body outside the catheter.

US5,578,006 describes a suction catheter for sucking mucus and other fluids from the tracheobronchial area of a patient. The catheter has a flexible tube with at least one lumen extended from a proximal end to a distal end. The lumen is provided with a funnel shaped enlarged outlet. EP2574364 which published on 3 April 2013 is therefore citable for novelty only under Article 54 (3) EPC describes a catheter having an elongated tubular body and a septum. The elongated tubular body includes a first lumen and a second lumen which communicate with respect to first and second distal openings. The septum separates the first and second lumens. One or both of the first and second walls includes a side opening in fluid communication with one of the first and second lumens. The side opening has an external aperture and an internal aperture. The internal aperture is smaller in dimension than the external aperture.

### SUMMARY OF THE INVENTION

The invention relates to a medical tube, as defined in claim 1, which is provided with a clog resistant orifice which exploits both the physics of the forces acting within a clogged orifice and the surrounding tube structure. The non-clogging or clog resistant orifices or holes according to the present disclosure minimize the occurrence of clogging and reduce the ability of clogs to block an orifice by one or more of the following: (i) eliminating tension and compression forces from forming within the hole, (ii) allowing the inner hole diameter to expand, which (a) can increase the pressure force exerted on the clog exponentially where P ∼ d² and (b) can cause shearing of the clog away from the wall when expansion occurs, and (iii) causing the wall to bend outward, away from the lumen when pressure is applied, or inward toward the lumen when suction is applied, which causes shearing of the clog away from the wall when this bending occurs. While the device disclosed herein find particular application for medical tubes, it may be useful and applied for other forms of tubing as well.

According to the invention a medical tube is provided, which is having reduced occurrence of clogging. The tube comprises a tubular wall having one or more clog resistant orifices. The tube will typically be flexible. The clog resistant orifices have an outwardly flared orifice wall so as to minimize the occurrence of clogging. The orifice wall may be flared outwardly at an angle of greater than 90 degrees or greater than or equal to 110 degrees, 130 degrees, 160 degrees, or even 170 degrees relative to the inner surface of the tubular wall. In some cases, the orifice wall may have an inner portion outwardly flared at a first angle of greater than 90 degrees relative to the inner surface of the tubular wall and an outer portion inwardly tapered at a different second angle of greater than 90 degrees relative to the outer surface of the tubular wall. For example, the first angle may be greater than or equal to 160 degrees and the second angle may be greater than or equal to 130 degrees. Typically, the clog resistant orifices will comprise an inner opening adjacent the inner surface of the tubular wall and an outer opening adjacent the outer surface of the tubular wall, with the outer opening being larger than the inner opening. The outwardly flaring or inwardly tapering shapes of the orifices can minimize tension and compression forces from forming within the orifice.

The portions of the tubular wall adjacent the orifices may be more flexible than the remainder of the tubular wall. According to the invention, these portions are configured to deform, for example by bending outwardly, when under fluid pressure from within the tube or bend inwardly when exposed to suction from within the tube. The one or more portions of the tubular wall adjacent the one or more orifices may be thinner than the remainder of the tubular wall. These structural properties can allow the inner hole diameter to expand or allow the wall to bend, facilitating the shearing of any clog away from the orifice wall.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1A shows a longitudinal section of a currently known and used medical tube with currently known and used transmission orifices or holes;
Figure 1B shows a magnified view of one of the clogged transmission orifices or holes in the medical tube of Figure 1A, including the forces acting on the clog, orifice, and surrounding wall;
Figure 2A shows a longitudinal section of a medical tube with clog resistant orifices according to embodiments of the present disclosure;
Figure 2B shows a magnified view of an un-clogged transmission orifice of the medical tube of Figure 2A;
Figure 2C shows a magnified view of a nearly clogged transmission orifice of the medical tube of Figure 2A, including the forces acting on the potential clog, orifice, and surrounding wall;
Figures 3A to 3C show a magnified view of a nearly clogged transmission orifice of a medical tube according to embodiments of the present disclosure as pressure is applied to the clog from fluid within the tube;
Figure 4A show an outside view of the outer surface of the medical tube with a clog resistant orifice, including the accumulation of a clog around the inner hole, according to embodiments of the disclosure;
Figure 4B show an outside view of the outer surface of the medical tube with a clog resistant orifice, including how the diameter of the inner hole expands when pressure is exerted, stretching the surface and causing shearing of the clog from the wall of the orifice, according to embodiments of the disclosure;
Figure 5A shows a magnified view of a clog resistant transmission orifice of a medical tube according to further embodiments of the present disclosure;
Figure 5B shows a magnified view of the clog resistant transmission orifice of Figure 5A with the inner wall bending outward when pressure is applied; and
Figure 5C shows a magnified view of the clog resistant transmission orifice of Figure 5A with the inner wall bending inward when pressure is applied.

### DETAILED DESCRIPTION OF THE INVENTION

Devices, apparatuses, systems, and methods are provided for a clog resistant orifice which exploits both the physics of the forces acting within a clogged orifice and the surrounding tube structure.

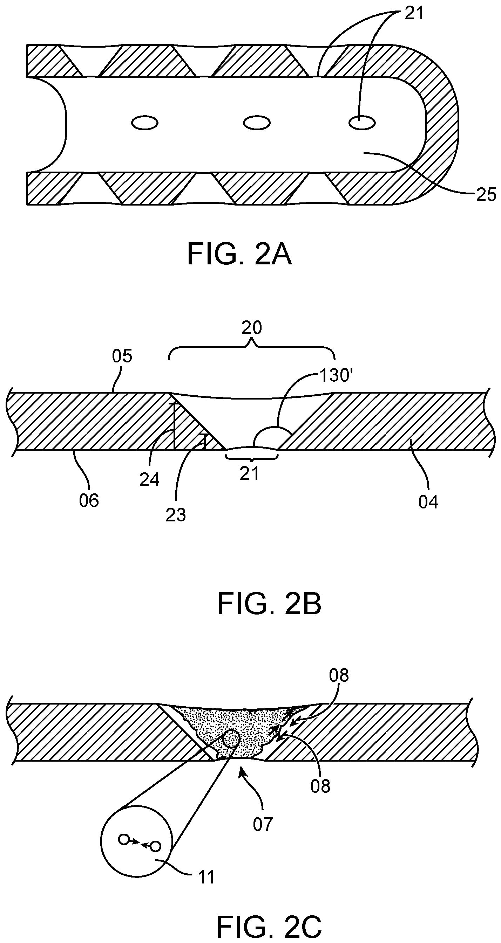

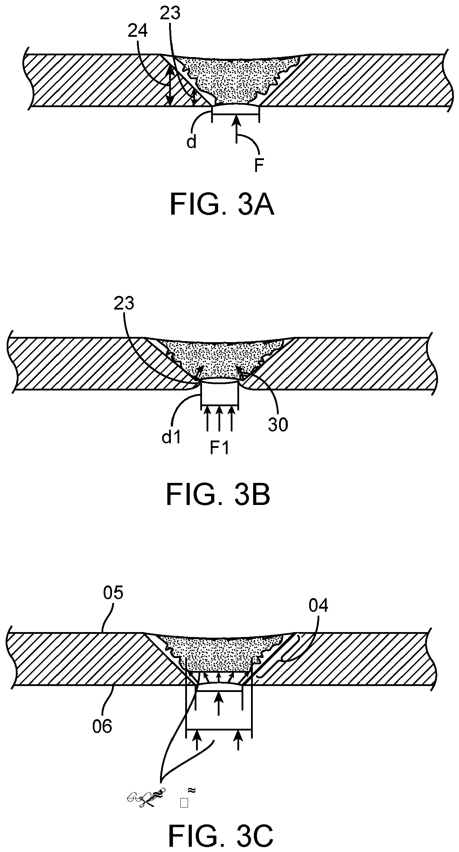

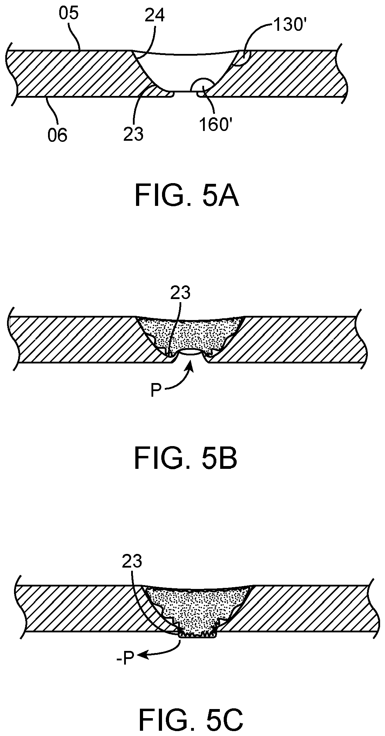

Figures 2A to 2B are illustrations of one embodiment of a clog resistant orifice. In this embodiment, a clog resistant orifice has an inner opening or hole **21** which opens into the lumen **25** of the device or tube. The inner hole is in fluid communication with an outer opening or hole **20** which opens into the outside of the tube, which, in some embodiments, would be in contact with a body cavity for which the tube is either instilling or draining a substance. In the present embodiment, the wall of the tube **04** is cut in a circular or oval shape with the outer wall **24** cut in a larger diameter than the inner wall **23** such that the angle of the wall forming the orifice is angled at greater than 90° in relation to the lumen surface **06** and outer tube surface **05.** In the embodiment of the present invention the angle is 130°, but this angle could vary from 110° to 170° within different embodiments. The angle within any embodiment would be sufficient to allow the inner wall to be thin enough to bend significantly outward (as with the clog resistant orifices shown, for example, by Figures 3B and 5B described below) when pressure or is applied from the inner lumen, and bend inward significantly (as with the clog resistant orifice shown, for example, by Figure 5C described below) when suction is applied from the inner lumen.

Figure 2A shows the end of a tube with several non-clogging holes. Figure 2B shows a close- up view of a clog resistant orifice which has formed a clog, including the various forces described above which may act on the clog. In this embodiment, the improved clog resistant orifice is shaped at an angle of 130° in relation to the inner wall **08,** with the inner opening or hole **21** being much smaller than the outer opening or hole **20.** The angle of the cut can serve several functions. First, since the wall of the hole is angled substantially greater than 90°, no tension or compression forces can occur which are greater than the adhesive force or cohesive forces, leaving only the adhesive forces **08** and cohesive forces **11** to overcome in order to clear the clog from the orifice. As shown in Figure 2C, the only forces acting to hold the clog in the hole are the adhesive forces between the wall and the clog and the cohesive forces holding the clog together. Secondly, since the thickness of the inner wall **23** within this embodiment is much thinner than the thickness at the outer wall **24,** the inner wall **23** bends outward when pressure is exerted by the fluid, and inward into the lumen when suction force is applied, which assists in forcing the clog off the wall by shearing action. The bending of the inner wall can also serve to enlarge the inner holes diameter, which increases the force applied to the clog as is illustrated in Figures 3A to 3C.

Figures 3A to 3C illustrate a method, not according to the invention, of clearing a clogged orifice whereby pressure or suction is applied to the inner lumen of a tube with clog resistant orifices. In Figure 3A, the inner hole has a diameter **d** when no pressure is applied to the lumen of the tube. In Figure 3B, a pressure force **F** on the inner hole bends the inner wall **23** outward and stretches the inner hole, making the making the diameter **d₁** larger. This allows for a larger surface area for the pressure force **F₁** to act on the clog. This larger surface area increases the force applied to the clog exponentially, where **F≈ d²,** and **F₁** ≈ **d₁².**

Figure 3A shows a pressure force **F** being applied by a fluid on the clog. The force is applied is proportional to the square of the diameter **d** of the hole at the lumen. When the fluid force is applied, the inner wall bends **23** in the direction of the clog, and this bending outward increases the diameter of the hole **D1,** which increases the force **F1** applied to the clog exponentially as shown in Figures 3B and 3C. In addition, the adhesive forces holding the clog in place adjacent to the opening of the hole at the inner lumen may be exposed to increased shearing forces **30** from the bending of the tube wall and the shearing forces thus created. These forces act to tear the clog away from the wall, which expose still more area for the pressure force to work against the adhesive and cohesive forces of the clog. Figure 3C shows how the clog has been broken away from the wall at the area immediately adjacent to the inner lumen and now the fluid can enter the hole further in, working on a larger area of the cohesive and adhesive forces of the clog.

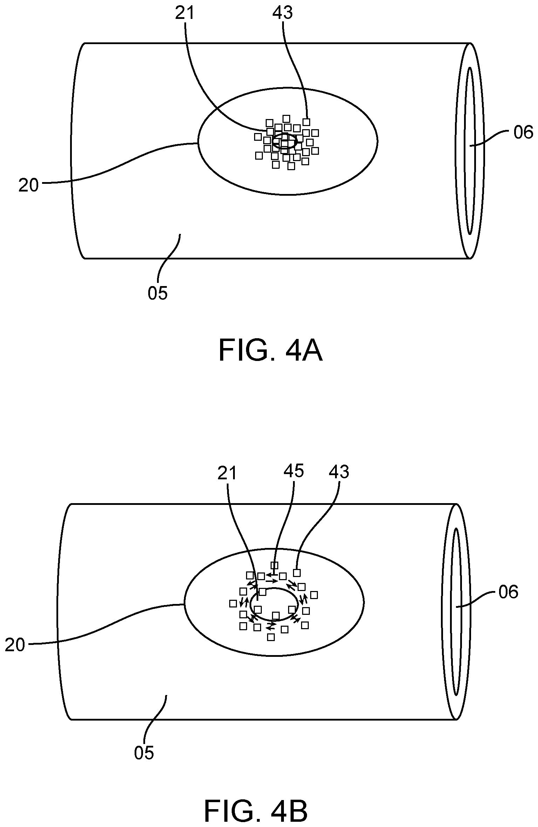

Figures 4A and 4B show an enlarged outside view of the outer wall surface **05** of a medical tube with a clog resistant orifice. Figure 4A shows how clogging particles **43** have formed around the inner hole **21** of the orifice. Figure 4B shows the same tubing segment with pressure applied to the inner lumen. Figure 4B illustrates how the increased diameter of the inner hole **21** created when fluid pressure is applied can cause a shearing **45** of the wall immediately surrounding the orifice by a stretching action when the inner hole expands. This stretching of the flexible material that makes up the wall of the orifice tears the particles from the wall and reopens the orifice.

As illustrated in Figure 4A and Figure 4B, methods disclosed herein further employ a shearing force **45,** created by the stretching and bending of the inner wall which acts to tear the clog from the wall. Figure 4A illustrates how particles **43** accumulate to form a clog on and around the inner hole of a clog resistant orifice. In Figure 4a, the inner hole **21** is completely clogged. In Figure 4B, pressure is applied to the inner lumen and the inner hole **21** stretches shearing away the particles and clearing the clog.

Lastly, the methods disclosed herein employ an angle of the orifice whereby the inner hole is smaller than the outer hole, and the wall is cut at an angle, which is substantially greater than 90° in relation to the inner lumen and outer tube wall. This angle serves to inhibit the formation of any substantial compression or tension forces within the hole.

Figures 5A to 5C are illustrations of an alternative embodiment of the present invention, whereby the thinness of the inner wall is maximized by increasing the angle at the inner wall **23** in relation to the outer wall **24** angle. Figure 5A shows a magnified view of a clog resistant transmission orifice of a medical tube according to further embodiments of the present disclosure, Figure 5B shows a magnified view of the clog resistant transmission orifice with the inner wall bending outward when pressure is applied, and Figure 5C shows a magnified view of the clog resistant transmission orifice of Figure 5C with the inner wall bending inward when pressure is applied. In the embodiment of Figures 5A to 5C, the angle close to the outer wall is 130°, and the angle close to the inner wall is 160°, but these angles may vary in different embodiments. One utility of this embodiment may be to maximize the angle of the inner hole while minimizing the size of the outer hole.

It will be apparent to a skilled artisan that the embodiments described herein are exemplary of the following claims that may have greater scope than any of the singular descriptions presented. There may be alterations made in these examples. For example, any portion of a clog resistant orifice may have varying shapes, angles, or designs within different embodiments and achieving the purpose of a clog resistant orifice as described in the present disclosure. The walls may be made of various materials or have any manner of coatings or treatments without departing from the scope of the following claims.

## Claims

1. A medical tube having reduced occurrence of clogging, the medical tube comprising:
a tubular wall (04) having one or more clog resistant orifices (20, 21), wherein the one or more clog resistant orifices have an outwardly flared orifice wall so as to minimize the occurrence of clogging for the one or more clog resistant orifices, **characterised in that** one or more portions of the tubular wall (04) adjacent the one or more clog resistant orifices (20, 21) are configured to deform under fluid pressure, facilitating shearing of any clog away from the one or more clog resistant orifices.

2. The medical tube of claim 1, wherein the tubular wall (04) has an inner surface and an outer surface (05), and wherein the one or more clog resistant orifices (20, 21) comprises an inner opening (21) adjacent the inner surface (06) of the tubular wall and an outer opening (20) adjacent the outer surface (05) of the tubular wall (04), the outer opening (20) being larger than the inner opening (21).

3. The medical tube of claim 1, wherein the tube is flexible.

4. The medical tube of claim 1, wherein one or more portions of the tubular wall (04) adjacent the one or more clog resistant orifices (20, 21) are more flexible than the remainder of the tubular wall.

5. The medical tube of claim 4, wherein the one or more portions of the tubular wall (04) adjacent the one or more clog resistant orifices (20, 21) is thinner than the remainder of the tubular wall.

6. The medical tube of claim 1, wherein one or more portions of the tubular wall (04) adjacent the one or more clog resistant orifices (20, 21) are configured to deform when under fluid pressure from within the tube.

7. The medical tube of claim 6, wherein the one or more portions of the tubular wall (04) adjacent the one or more clog resistant orifices (20, 21) are configured to bend outwardly when under fluid pressure from within the tube.

8. The medical tube of claim 1, wherein one or more portions of the tubular wall (04) adjacent the one or more clog resistant orifices (20, 21) are configured to deform when under suction from within the tube.

9. The medical tube of claim 8, wherein the one or more portions of the tubular wall (04) adjacent the one or more clog resistant orifices (20, 21) are configured to bend inwardly when exposed to suction from within the medical tube.

10. The medical tube of claim 1, wherein the tubular wall (04) has an inner surface (06) and the orifice wall is flared outwardly at an angle of greater than 90 degrees relative to the inner surface (06) of the tubular wall (04).

11. The medical tube of claim 10, wherein the orifice wall is flared outwardly at an angle of greater than or equal to 110 degrees relative to the inner surface (06) of the tubular wall (04).

12. The medical tube of claim 11, wherein the orifice wall is flared outwardly at an angle of greater than or equal to 130 degrees relative to the inner surface (06) of the tubular wall (04).

13. The medical tube of claim 1, wherein the tubular wall has an inner surface (06) and an outer surface (05), and wherein the orifice wall of the one or more clog resistant orifices (20, 21) comprises an inner portion outwardly flared a first angle of greater than 90 degrees relative to the inner surface (06) of the tubular wall and an outer portion (05) inwardly tapered at a second angle of greater than 90 degrees relative to the outer surface of the tubular wall.

14. The medical tube of claim 13, wherein the first angle is greater than or equal to 160 degrees and the second angle is greater than or equal to 130.

## Patentansprüche

1. Ein medizinischer Schlauch mit einem verringerten Auftreten von Verstopfungen, wobei der medizinische Schlauch Folgendes beinhaltet:
eine röhrenförmige Wand (04) mit einer oder mehreren verstopfungssicheren Öffnungen (20, 21), wobei die eine oder mehreren verstopfungssicheren Öffnungen eine nach außen konisch aufgeweitete Öffnungswand aufweisen, um somit das Auftreten von Verstopfungen für die eine oder mehreren verstopfungssicheren Öffnungen auf ein Mindestmaß zu verringern, **dadurch gekennzeichnet, dass** ein oder mehrere Anteile der röhrenförmigen Wand (04) neben der einen oder den mehreren verstopfungssicheren Öffnungen (20, 21) dazu ausgelegt sind, sich unter Fluiddruck zu verformen, was ein leichteres Abscheren einer Verstopfung von der einen oder den mehreren verstopfungssicheren Öffnungen ermöglicht.

2. Medizinischer Schlauch nach Anspruch 1, wobei die röhrenförmige Wand (04) eine innere Oberfläche und eine äußere Oberfläche (05) aufweist und wobei die eine oder mehreren verstopfungssicheren Öffnungen (20, 21) ein inneres Durchgangloch (21) neben der inneren Oberfläche (06) der röhrenförmigen Wand und ein äußeres Durchgangsloch (20) neben der äußeren Oberfläche (05) der röhrenförmigen Wand (04) beinhalten, wobei das äußere Durchgangsloch (20) größer als das innere Durchgangsloch (21) ist.

3. Medizinischer Schlauch nach Anspruch 1, wobei der Schlauch biegsam ist.

4. Medizinischer Schlauch nach Anspruch 1, wobei ein oder mehrere Anteile der röhrenförmigen Wand (04) neben der einen oder den mehreren verstopfungssicheren Öffnungen (20, 21) biegsamer als die restliche röhrenförmige Wand ist.

5. Medizinischer Schlauch nach Anspruch 4, wobei der eine oder die mehreren Anteile der röhrenförmigen Wand (04) neben der einen oder den mehreren verstopfungssicheren Öffnungen (20, 21) dünner als die übrige röhrenförmige Wand ist.

6. Medizinischer Schlauch nach Anspruch 1, wobei ein oder mehrere Anteile der röhrenförmigen Wand (04) neben der einen oder den mehreren verstopfungssicheren Öffnungen (20, 21) dazu ausgelegt sind, sich unter Fluiddruck aus dem Inneren des Schlauchs zu verformen.

7. Medizinischer Schlauch nach Anspruch 6, wobei der eine oder die mehreren Anteile der röhrenförmigen Wand (04) neben der einen oder den mehreren verstopfungssicheren Öffnungen (20, 21) dazu ausgelegt sind, sich unter Fluiddruck aus dem Inneren des Schlauchs nach außen zu biegen.

8. Medizinischer Schlauch nach Anspruch 1, wobei ein oder mehrere Anteile der röhrenförmigen Wand (04) neben der einen oder den mehreren verstopfungssicheren Öffnungen (20, 21) dazu ausgelegt sind, sich unter Saugwirkung aus dem Inneren des Schlauchs zu verformen.

9. Medizinischer Schlauch nach Anspruch 8, wobei der eine oder die mehreren Anteile der röhrenförmigen Wand (04) neben der einen oder den mehreren verstopfungssicheren Öffnungen (20, 21) dazu ausgelegt sind, sich nach innen zu biegen, wenn sie einer Saugwirkung aus dem Inneren des medizinischen Schlauchs ausgesetzt sind.

10. Medizinischer Schlauch nach Anspruch 1, wobei die röhrenförmige Wand (04) eine innere Oberfläche (06) aufweist und die Öffnungswand in einem Winkel von mehr als 90 Grad relativ zu der inneren Oberfläche (06) der röhrenförmigen Wand (04) konisch nach außen aufgeweitet ist.

11. Medizinischer Schlauch nach Anspruch 10, wobei die Öffnungswand in einem Winkel von mehr als oder gleich 110 Grad relativ zu der inneren Oberfläche (06) der röhrenförmigen Wand (04) konisch nach außen aufgeweitet ist.

12. Medizinischer Schlauch nach Anspruch 11, wobei die Öffnungswand in einem Winkel von mehr als oder gleich 130 Grad relativ zu der inneren Oberfläche (06) der röhrenförmigen Wand (04) konisch nach außen aufgeweitet ist.

13. Medizinischer Schlauch nach Anspruch 1, wobei die röhrenförmige Wand eine innere Oberfläche (06) und eine äußere Oberfläche (05) aufweist und wobei die Öffnungswand der einen oder mehreren verstopfungssicheren Öffnungen (20, 21) einen inneren Anteil, der in einem ersten Winkel von mehr als 90 Grad relativ zu der inneren Oberfläche (06) der röhrenförmigen Wand konisch nach außen aufgeweitet ist, und einen äußeren Anteil (05), der in einem zweiten Winkel von mehr als 90 Grad relativ zu der äußeren Oberfläche der röhrenförmigen Wand nach innen verjüngt ist, beinhaltet.

14. Medizinischer Schlauch nach Anspruch 13, wobei der erste Winkel größer als oder gleich 160 Grad ist und der zweite Winkel größer als oder gleich 130 Grad ist.

## Revendications

1. Tube médical ayant une apparition réduite d'obstruction, le tube médical comprenant :
une paroi tubulaire (04) ayant un ou plusieurs orifices résistant à l'obstruction (20, 21), dans lequel les un ou plusieurs orifices résistant à l'obstruction ont une paroi d'orifice évasée vers l'extérieur de manière à minimiser l'apparition d'obstruction pour les un ou plusieurs orifices résistant à l'obstruction, **caractérisé en ce qu'**une ou plusieurs parties de la paroi tubulaire (04) adjacentes aux un ou plusieurs orifices résistant à l'obstruction (20, 21) sont configurées pour se déformer sous pression de fluide, facilitant le cisaillement de toute obstruction pour l'éloigner des un ou plusieurs orifices résistant à l'obstruction.

2. Tube médical selon la revendication 1, dans lequel la paroi tubulaire (04) a une surface interne et une surface externe (05), et dans lequel les un ou plusieurs orifices résistant à l'obstruction (20, 21) comprennent une ouverture interne (21) adjacente à la surface interne (06) de la paroi tubulaire et une ouverture externe (20) adjacente à la surface externe (05) de la paroi tubulaire (04), l'ouverture externe (20) étant plus large que l'ouverture interne (21).

3. Tube médical selon la revendication 1, dans lequel le tube est flexible.

4. Tube médical selon la revendication 1, dans lequel une ou plusieurs parties de la paroi tubulaire (04) adjacentes aux un ou plusieurs orifices résistant à l'obstruction (20, 21) sont plus flexibles que le reste de la paroi tubulaire.

5. Tube médical selon la revendication 4, dans lequel les une ou plusieurs parties de la paroi tubulaire (04) adjacentes aux un ou plusieurs orifices résistant à l'obstruction (20, 21) sont plus fines que le reste de la paroi tubulaire.

6. Tube médical selon la revendication 1, dans lequel une ou plusieurs parties de la paroi tubulaire (04) adjacentes aux un ou plusieurs orifices résistant à l'obstruction (20, 21) sont configurées pour se déformer quand elles sont sous pression de fluide de l'intérieur du tube.

7. Tube médical selon la revendication 6, dans lequel les une ou plusieurs parties de la paroi tubulaire (04) adjacentes aux un ou plusieurs orifices résistant à l'obstruction (20, 21) sont configurées pour plier vers l'extérieur quand elles sont sous pression de fluide de l'intérieur du tube.

8. Tube médical selon la revendication 1, dans lequel une ou plusieurs parties de la paroi tubulaire (04) adjacentes aux un ou plusieurs orifices résistant à l'obstruction (20, 21) sont configurées pour se déformer quand elles sont sous aspiration de l'intérieur du tube.

9. Tube médical selon la revendication 8, dans lequel les une ou plusieurs parties de la paroi tubulaire (04) adjacentes aux un ou plusieurs orifices résistant à l'obstruction (20, 21) sont configurées pour plier vers l'intérieur quand elles sont exposées à l'aspiration de l'intérieur du tube médical.

10. Tube médical selon la revendication 1, dans lequel la paroi tubulaire (04) a une surface interne (06) et la paroi d'orifice est évasée vers l'extérieur à un angle supérieur à 90 degrés par rapport à la surface interne (06) de la paroi tubulaire (04).

11. Tube médical selon la revendication 10, dans lequel la paroi d'orifice est évasée vers l'extérieur à un angle supérieur ou égal à 110 degrés par rapport à la surface interne (06) de la paroi tubulaire (04).

12. Tube médical selon la revendication 11, dans lequel la paroi d'orifice est évasée vers l'extérieur à un angle supérieur ou égal à 130 degrés par rapport à la surface interne (06) de la paroi tubulaire (04).

13. Tube médical selon la revendication 1, dans lequel la paroi tubulaire a une surface interne (06) et une surface externe (05), et dans lequel la paroi d'orifice des un ou plusieurs orifices résistant à l'obstruction (20, 21) comprend une paroi interne évasée vers l'extérieur à un premier angle supérieur à 90 degrés par rapport à la surface interne (06) de la paroi tubulaire et une partie externe (05) effilée vers l'intérieur à un deuxième angle supérieur à 90 degrés par rapport à la surface externe de la paroi tubulaire.

14. Tube médical selon la revendication 13, dans lequel le premier angle est supérieur ou égal à 160 degrés et le deuxième angle est supérieur ou égal à 130.
